(19) Europäisches Patentamt
European Patent Office
Office européen des brevets

(11) Veröffentlichungsnummer: **0 593 425 B1**

(12) # EUROPÄISCHE PATENTSCHRIFT

(45) Veröffentlichungstag der Patentschrift: **28.12.94**

(51) Int. Cl.5: **B01J 31/06**, B01J 23/46, C07C 29/48

(21) Anmeldenummer: **90909659.6**

(22) Anmeldetag: **16.06.90**

(86) Internationale Anmeldenummer: **PCT/EP90/00954**

(87) Internationale Veröffentlichungsnummer: **WO 91/00143 (10.01.91 91/02)**

(54) **OXIDISCHE HETEROGENKATALYSATOREN DES OSMIUMS, VERFAHREN ZU IHRER HERSTELLUNG UND IHRE VERWENDUNG FÜR DIE ÜBERFÜHRUNG VON OLEFINISCHEN VERBINDUNGEN IN GLYKOLE.**

(30) Priorität: **26.06.89 DE 3920917**

(43) Veröffentlichungstag der Anmeldung: **27.04.94 Patentblatt 94/17**

(45) Bekanntmachung des Hinweises auf die Patenterteilung: **28.12.94 Patentblatt 94/52**

(84) Benannte Vertragsstaaten: **BE CH DE FR GB IT LI**

(56) Entgegenhaltungen:
**EP-A- 32 318**
**EP-A- 77 202**
**EP-A- 246 031**

**Synthesis, Journal of Synthetic Org. Chem. ,no.1, January 1989, Stuttgart,DE, G. Cainelli et al.: " Catalytic hydroxylation of olefins by polymer-bound osmium tetroxide," Seiten 45-47**

(73) Patentinhaber: **HOECHST AKTIENGESELLSCHAFT**
**D-65926 Frankfurt (DE)**

(72) Erfinder: **HERRMANN, Wolfgang, A.**
**Waldweg 10**
**D-8051 Giggenhausen (DE)**
Erfinder: **WEICHSELBAUMER, Georg**
**Richildisstrasse 6**
**D-8899 Hohenwart (DE)**

**EP 0 593 425 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Vorliegende Erfindung bezieht sich auf oxidische Heterogenkatalysatoren des Osmiums, Verfahren zu ihrer Herstellung durch Umsetzung von löslichen Osmiumverbindungen, vorzugsweise $OsO_4$ und $Os_3(CO)_{12}$, mit als Trägermaterialien geeigneten, reaktive Gruppen enthaltenden Polymeren sowie auf die Verwendung dieser Katalysatoren für die Oxidation von olefinischen Verbindungen zu Glykolen (vicinalen Diolen), insbesondere zur stereospezifischen cis-Hydroxylierung von Olefinen, deren Doppelbindung sich im Ring befindet, mittels Wasserstoffperoxid.

Es ist bekannt, daß Osmiumtetroxid ($OsO_4$) auf ungesättigte organische Verbindungen oxidierend wirkt oder die Oxidation solcher Verbindungen durch andere "primäre" Oxidationsmittel wie tert.-Butylhydroperoxid, Natriumchlorat, Natriumhypochlorit, N-Methylmorpholin-N-oxid, Trimethylamin-N-oxid sehr wirksam katalysiert. Besonders beliebt ist Osmiumtetroxid als Oxidationsmittel bzw. als Katalysator für die stereospezifische Überführung von Olefinen in cis-Glykole, obwohl die selektivitätsmindernde Weiteroxidation der Glykole zu $\alpha$-Ketolen eine störende Nebenreaktion sein kann. Solche Sekundäroxidationen sind bei Verwendung von Wasserstoffperoxid am stärksten ausgeprägt, so daß dieses Oxidationsmittel in der Katalysechemie des Osmiums bisher ohne Bedeutung ist.

Es gibt andererseits keinen Katalysator außer Osmiumtetroxid, mit dessen Hilfe die unmittelbare Überführung von Olefinen in cis-Glykole möglich ist. Auch nahe verwandte Metalloxide und Oxometallate zeigen ein abweichendes Reaktionsverhalten: So spaltet Rutheniumtetroxid ($RuO_4$) Olefine in Ketone unter Bruch der olefinischen Doppelbindung; alkalisches Permanganat ($[MnO_4]^-$) ist weit weniger selektiv als Osmiumtetroxid (R. A. Sheldon und J. K. Kochi: "Metal Catalyzed Oxidation of Organic Compounds", Academic Press, New York, London, Toronto, Sydney, San Fransisco 1981, 162-166, 179-188, 294-296. Perrhenat ($[ReO_4]^-$) ist nach eigenen Versuchen vollkommen unwirksam.

Trotz dieser Vorteile weist das Osmiumtetroxid zwei prinzipielle, gravierende Nachteile auf:

1) Es ist extrem toxisch und wegen seiner hohen Flüchtigkeit (Fp. 40 °C; Kp. 132 °C) besonders gefährlich. Für präparative und katalytische Anwendungen ist das Ausmaß der Toxizität prohibitiv. Es verursacht Bindehautentzündungen sowie Trübungen der Hornhaut und kann sowohl durch Kontakt mit der Haut (insbesondere in Lösung) als auch durch Inhalation in den Körper gelangen. Es weist mit Beryllium und dessen Verbindungen den niedrigsten MAK-Wert (= Maximale Arbeitsplatzkonzentration und biologische Arbeitsstofftoleranz) (0,002 mg/m$^3$) Unter allen anorganischen Verbindungen auf (Römpps Chemielexikon, 8. Aufl., 4, 2930-31, Franckh'sche Verlagshandlung, Stuttgart (1985); D. Hunter, J. Pharm. Pharmacol. 5 (1953) 149-150; D. Hunter, Brit. Med. Bull. 7 (1950) 11; A. I. G. McLaughlin et al., Brit. J. Ind. Med. 3 (1946) 183-186).

2) Wird Osmiumtetroxid als Oxidationskatalysator für olefinische Verbindungen eingesetzt, so bedarf es zusätzlich spezieller ("primärer") Oxidationsmittel: tert.-Butylhydroperoxid ist am gebräuchlichsten; das billigere Wasserstoffperoxid eignet sich deshalb nicht, weil, wie oben angegeben, die damit erreichbaren Selektivitäten an gewünschtem Produkt nicht hoch genug sind und eine Weiteroxidation zum Ketol erfolgt.

Die Verwendung von Osmiumtetroxid ist deshalb auf die stereospezifische cis-Hydroxylierung kleiner Mengen präparativ wertvoller Olefine im Laboratoriumsmaßstab beschränkt; im technischen Maßstab spielt Osmiumtetroxid keine Rolle.

Es besteht daher seit langem die Aufgabe, das Osmiumtetroxid unter Wahrung seiner hervorragenden katalytischen Eigenschaften chemisch so zu modifizieren, daß seine akute Giftigkeit beseitigt oder zumindest stark eingeschränkt ist; um eine wirtschaftliche technische Nutzung zu ermöglichen, muß darüber hinaus das überwiegend verwendete, da mit Abstand selektivste, "primäre" Oxidationsmittel tert.-Butylhydroperoxid durch billigere Substanzen ersetzt werden, möglichst durch Wasserstoffperoxid.

In der Vergangenheit hat es nicht an Versuchen gefehlt, die vorstehend aufgeführten Probleme zu lösen. Einer Beobachtung von Criegee et al. (Liebigs Ann. Chem. 550 (1942) 99-100) und Griffith et al. (J. Chem. Soc. Dalton 1977, 941-944) folgend, hat man Amin-Basen (L), insbesondere tertiäre Amine wie Pyridin $C_5H_5N$, Chinuclidin $C_7H_{13}N$ und 1,4-Diazabicyclo[2,2,2]octan $C_6H_{12}N_2$ ("Dabco") an das Osmiumtetroxid gebunden. Die gebildeten Komplexe der Formel $OsO_4 \cdot L$ sind zwar weniger flüchtig und katalytisch sogar aktiver als $OsO_4$ selbst, sind aber weder bei höheren Temperaturen ausreichend stabil noch dauerhaft stabil gegen die gewöhnlich verwendeten Oxidationsmittel. Es ist also durch diese Maßnahme nicht sicher genug auszuschließen, daß in den Reaktionsansätzen immer noch freies $OsO_4$ vorhanden ist. Hieran ändert sich auch nichts, wenn $OsO_4$ nicht an monomere, sondern an polymere Basen desselben Typs über die tertiären Stickstoffatome gebunden wird, etwa an Poly(4-vinylpyridin) und analoge Polymere (G. Cainelli et al., Synthesis 1989, 45-47). In solchem, aus einer Cyclohexan-Lösung auf den polymeren Träger niedergeschlagenem und an diesen gebundenem $OsO_4$ sind die Aktivität und Selektivität der bereits

bekannten Komplexe $OsO_4 \cdot L$ (L = z.B. Pyridin) unverändert erhalten. Es wirkt also in ausreichender Aktivität und Selektivität nur mit tert.-Butylhydroperoxid bei Raumtemperatur oder mit Trimethylamin-N-oxid bei 83 °C, in beiden Fällen in tert.-Butanol als Lösungsmittel (Cainelli et al., l.c.). Wendet man $H_2O_2$ als primäres Oxidationsmittel an, so ist die Selektivität der Bildung von Glykolen ähnlich schlecht wie bei freiem $OsO_4$ und den Komplexen $OsO_4 \cdot L$, und es findet Ketol-Bildung statt (vgl. Cainelli et al., l.c.).

Cainelli et al., l.c., beschreiben zwei Varianten zur Herstellung von Katalysatoren, die $OsO_4$ auf polymeren Trägern enthalten. Nach einer Methode wurde in siedendem Chloroform (24 Stunden) ein tertiäres Diamin (z.B. 1,4-Diazabicyclo[2,2,2]octan) mit chlormethyliertem Styrol/Divinylbenzol-Copolymeren ("Merrifield- Harz") quaternisiert und dann mit $OsO_4$ beladen. Nach der anderen Methode wurde Poly(4-vinylpyridin) mit $OsO_4$ in Cyclohexan bei Raumtemperatur in einer Inertgasatmosphäre über Nacht behandelt. Wenn man nach dieser Methode in aliphatischen Kohlenwasserstofflösungsmitteln, wie in Cyclohexan arbeitet, so erhält man unabhängig von der Reaktionszeit ausschließlich eine gelbe Form des Polymeren, die das $OsO_4$ im Vakuum leicht abspaltet, z.B. bei 60 °C und $10^{-5}$ Pa.

Eigene Versuche haben gezeigt, daß das von Cainelli et al. beschriebene, an Polymere gebundene Osmiumtetroxid im Hochvakuum bei nur geringfügig erhöhter Temperatur (ab ca. 60 °C) wieder als solches vom Trägermaterial absublimiert. Das Infrarotspektrum des in Cyclohexan beladenen polymeren Trägers, aufgenommen mit der Technik der diffusen Reflexion, zeigt die charakteristische intensive Bande bei 907 $cm^{-1}$, die für OsO-Valenzschwingungen von intaktem, an ein koordinierendes Stickstoffatom gebundenem Osmiumtetroxid typisch ist (Fig. 1). Die Bindung des Osmiumtetroxids an diese Polymeren ist also nicht sehr stark und sie ist außerdem - wie von Cainelli et al. angenommen wird - auf die tertiären Stickstoffatome des Trägers beschränkt. Eine andauernde Haftung des Katalysators ist also durch diese Maßnahme grundsätzlich nicht erreichbar.

Die oben formulierten Aufgaben wurden jetzt erfindungsgemäß dadurch gelöst, daß ein Osmiumoxid auf ein stickstoffhaltiges Polymeres aufgebracht oder dort erzeugt wird. Dazu muß ein Polymeres verwendet werden, das einerseits zur primären Osmium-Fixierung ausreichend basische Zentren besitzt und das andererseits mit dem so an das Polymere herangeführten oder dort z.B. aus $(Os_3(CO)_{12})$ oxidativ erzeugtem Osmiumoxid unter irreversibler Verankerung reagiert.

Gegenstand der Erfindung sind also oxidische Heterogen-Katalysatoren auf der Grundlage von stickstoffhaltigen Polymeren, an die oxidisches Osmium in einer niedrigeren als der achtwertigen Oxidationsstufe gebunden ist.

Gegenstand der Erfindung ist auch ein Verfahren zur Herstellung von Katalysatoren auf der Grundlage von stickstoffhaltigen Polymeren, auf die ein Osmiumoxid aus einem Lösungsmittel niedergeschlagen ist, das dadurch gekennzeichnet ist, daß man ein in Alkoholen und/oder Äthern lösliches Osmiumoxid in Gegenwart eines solchen Alkohols und/oder Äthers mit einem in Wasser und dem angewandten Alkohol und Äther unlöslichen Polymeren, das Stickstoff in offenkettiger, heterocyclischer und/oder Amidbindung bzw. ein davon abgeleitetes N-Oxid enthält, unter Bedingungen, unter denen die achtwertige Oxidationsstufe des Osmiums nicht beständig ist, umsetzt und den oxidisches Osmium enthaltenden Katalysator abtrennt und trocknet.

Gegenstand der Erfindung ist auch ein Verfahren zur Herstellung von Katalysatoren auf der Grundlage von stickstoffhaltigen Polymeren, auf die eine Osmiumverbindung aus einem Lösungsmittel niedergeschlagen ist, das dadurch gekennzeichnet ist, daß man eine Osmium-Carbonyl-Verbindung in einem Alkohol und/oder Äther mit einem in Wasser und dem angewandten Alkohol und Äther unlöslichen Polymeren, das Stickstoff in offenkettiger, heterocyclischer und/oder Amidbindung bzw. ein davon abgeleitetes N-Oxid enthält, unter Bedingungen umsetzt, die zu einer Fixierung eines Osmiumcarbonyls oder eines CO-haltigen Bruchstücks der Carbonylosmium-Verbindung auf dem Polymeren führt und das in dem so erhaltenen Produkt enthaltene Osmium mit einer Peroxyverbindung, wie Salze von anorganischen Peroxysäuren, wie Ammoniumperoxodisulfat, insbesondere $H_2O_2$, zu einer höheren, jedoch nicht der achtwertigen Oxidationsstufe oxidiert.

Als Alkohole kommen insbesondere einwertige aliphatische Alkohole mit 1 bis 5 C-Atomen in Frage, die geradkettig oder verzweigt sein können und die Hydroxylgruppe an einem primären, sekundären oder tertiären C-Atom tragen, wie Methanol, Äthanol und die verschiedenen Propanole, Butanole und Pentanole, wie n- oder Isopropanol, n-Butanol oder Amylalkohol. Geeignete Äther sind die von den vorgenannten Alkoholen abgeleiteten Äther, insbesondere Diäthyl-, Diisopropyl, Di- und butyläther, ferner cyclische Äther wie Tetrahydrofuran und Dioxan.

Weitere Gegenstände der Erfindung bestehen in der Verwendung der erfindungsgemäßen Heterogenkatalysatoren zur Hydroxylierung von olefinischen Bindungen zu Glykolen und in einem Verfahren zur Hydroxylierung von olefinischen Bindungen zu Glykolen, das dadurch gekennzeichnet ist, daß man an solchen Katalysatoren olefinische Verbindungen der Formel $R^1R^2C=CR^3R^4$, in denen $R^1$ bis $R^4$ gleich oder

verschieden sind, wobei einer oder zwei dieser Reste aromatisch sein können, die übrigen aber nicht-aromatische Reste bedeuten, mit Wasserstoffperoxid, das in einem von aliphatischen Kohlenwasserstoffen verschiedenen Lösungsmittel gelöst ist, an den erfindungsgemäßen oxidischen Heterogenkatalysatoren umsetzt (Beispiele 4 bis 45).

Wenn man olefinische Verbindungen oxidiert, deren Doppelbindungen sich in einem Ring befinden, erhält man bekanntlich Glykole (cis-Diole), in denen die Drehbarkeit der C-C-Bindungen zwischen den vicinalen Hydroxylgruppen eingeschränkt ist. Es ist ein Vorteil der vorliegenden Erfindung, daß sich solche olefinische Bindungen mit Hilfe der erfindungsgemäßen Katalysatoren stereospezifisch cis-hydroxylieren lassen.

An die Konstitution der als Ausgangsstoff verwendeten Olefine $R^1R^2C=CR^3R^4$ sind keine Bedingungen gestellt, außer daß sie sterisch nicht überladen sein dürfen. Bei Verwendung vierfach substituierter Olefine, in denen also $R^1$, $R^2$, $R^3$ und $R^4$ eine andere Bedeutung als Wasserstoff haben, gehen die tatsächlichen Umsätze etwas zurück. Die nichtaromatischen Reste bedeuten also aliphatische Reste und sind acyclisch oder cyclisch. Sie können auch eine olefinische Gruppierung enthalten, die reaktiv sein kann oder unter den angewandten Bedingungen recht beständig ist, ferner eine Cyanogruppe und/oder ein Carbonylderivat, z.B. eine Aldehyd, Keto-, Carboxyl oder Estergruppe, wobei die CO-Gruppe direkt an die C=C-Doppelbindung gebunden sein kann. Die Reste können auch Halogen enthalten, soweit dieses so fest gebunden ist, daß es unter den Reaktionsbedingungen nicht hydrolysiert oder oxidiert wird, also z.B. Fluor, Chlor oder Brom (Beispiel 16). Geeignetes Ausgangsmaterial ist z.B. Allylbromid. Zweckmäßig ist wenigstens der Rest $R^1$ Wasserstoff und vorzugsweise auch ein weiterer der Reste $R^2$ bis $R^4$. Die Reste $R^2$ und $R^3$ bzw. $R^4$ können auch miteinander verbunden sein, z.B. in cyclischen Olefinen. Geeignet sind auch Verbindungen mit einer exocyclischen Doppelbindung, in denen also eine $=CH_2$-Gruppe an ein cyclisches C-Atom, z.B. des Cyclohexans, gebunden ist (Beispiel 21).

Die polymeren Träger können primäre, sekundäre oder tertiäre Amin-Baugruppen (acyclisch oder cyclisch), organische Aminoxid-, Amid- oder Imid-Funktionen oder stickstoffhaltige Heterocyclen beliebiger Größe (z.B. Pyridyl, Imidazyl usw.) enthalten. Es ist unwesentlich, welche Molmassen die verwendbaren Polymeren haben, solange sie in Wasser sowie in dem angewandten Alkohol und Äther unlöslich sind. Besonders bewährt haben sich die im Handel verfügbaren Polymeren

a) Reillex 402 Poly(4-vinylpyridin), quervernetzt, Nr. 22,696-3, Aldrich Chemical Co., USA,

b) Reilline 2202 Poly(2-vinylpyridin) mit einer mittleren Molmasse von 200000, Nr. 30,521-9, Aldrich Chemical Co., USA,

c) Poly(4-vinylpyridin-co-styrol) mit 10 % Styrol-Gehalt, Nr. 19,207-4, Aldrich Chemical Co., USA,

d) Poly(4-vinylpyridin-co-methacrylsäurebutylester) mit 10 % Butylmethacrylat, Nr. 30,625-8, Aldrich Chemical Co., USA,

e) Poly(4-vinylpyridin), vernetzt mit 2 % Divinylbenzol, Nr. 81391 der Fluka AG, Buchs, Schweiz,

f) Polyimid, Aldrich Nr. 18,464-0, Aldrich Chemical Co., USA,

g) Poly(N-vinylcarbazol), Nr. 18,260-5, Aldrich Chemical Co., USA,

h) die sich von a - e) durch Oxidation ableitenden N-Oxide, z.B. das aus a) und e) erhältliche Poly(4-vinylpyridin-N-oxid) gemäß Beispiel 2, sowie

i) Polymere mit primären, sekundären und/oder tertiären Amingruppen, die über n-Propylreste mit $Si(OR)_3$-Einheiten verknüpft sind, wie solche der Fa. Degussa AG, die sich von den folgenden Formeln ableiten

$i_1$) $H_2N(CH_2)_3Si(OC_2H_5)_3$

$i_2$) $H_2N(CH_2)_2NH(CH_2)_3Si(OC_2H_5)_3$

$i_3$) $N[CH_2CH_2CH_2Si(OC_2H_5)_3]_3$

$i_4$) $HN[CH_2CH_2CH_2Si(OC_2H_5)_3]_2$

Die aus den unter $i_1$) bis $i_4$) durch Hydrolyse entstehenden Polymeren verhalten sich unter den Oxidationsbedingungen praktisch identisch. Es sind deshalb in den Beispielen 28 - 45 Umsetzungen unter Verwendung von nur zwei polymeren Trägern ($i_3$ und $i_4$) angegeben.

Bei den unter i) genannten polymeren Trägern handelt es sich um primäre, sekundäre und tertiäre Aminverbindungen, die über n-Propylenreste mit $-Si(OR)_3$-Einheiten verknüpft sind; bei Hydrolyse dieser Si-Reste vollzieht sich die Polymerisation, also z.B. folgendermaßen

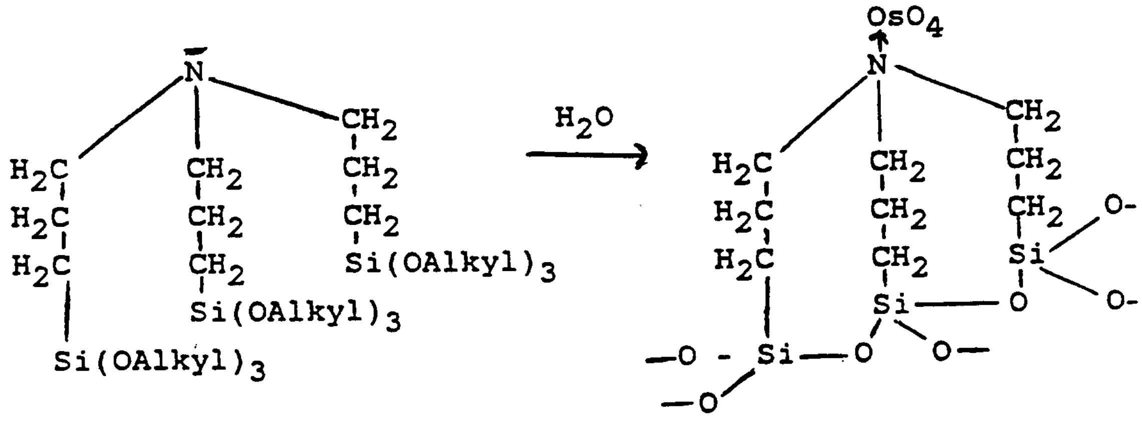


Geeignete Katalysatoren lassen sich z.B. aus den Polymeren a) bis i) folgendermaßen herstellen:

Man läßt auf das betreffende Polymere einige Tage, z.B. 3 Tage bei Raumtemperatur eine Lösung von $OsO_4$ in absolutem Tetrahydrofuran einwirken, vorteilhaft unter inniger Durchmischung des Reaktionssystems, z.B. durch Rühren. Wenn man Äther oder Alkohole mit mehr als 3 C-Atomen verwendet, läßt sich die Reaktionszeit jedoch ganz erheblich, und zwar bei Raumtemperatur auf z.B. 2 h abkürzen, wenn man kurze Zeit, z.B. bereits eine Stunde nach Beginn der Reaktion, ein stärker reduzierend wirkendes Mittel zugibt. Als solche eignen sich z.B. Hydrazin und einwertige aliphatische Alkohole mit 1 bis 6, insbesondere mit 1 bis 3 C-Atomen und vor allem Methanol und Äthanol. Die Zugabe solcher reduzierend wirkender Mittel ist vor allem dann von Bedeutung, wenn die Reaktion in einem Äther oder in einem verhältnismäßig schwach reduzierend wirkenden Alkohol durchgeführt wird. Zweckmäßig fügt man der Lösung 2 bis 20, vorzugsweise 3 bis 6 Mol des Alkohols je Mol $OsO_4$ zu. Dann kann das Unlösliche vom Lösungsmittel abfiltriert werden. Dieses ist als solches als Katalysator geeignet. Man kann aber auch das Unlösliche einen Tag in einer Lösung von $H_2O_2$ in einem organischen Lösungsmittel, wie sie weiter unten und z.B. im Beispiel 1b beschrieben wird, rühren, dann abfiltrieren, im Vakuum einer Ölpumpe trocknen und in dieser Form verwenden. Statt der chemischen Reduktion kann mit gleichem Effekt eine Behandlung mit Ultraschall stattfinden.

Eine andere Methode besteht darin, daß man eine Suspension des betreffenden Polymeren in einer Lösung von Dodecacarbonyltriosmium $Os_3(CO)_{12}$ in einem Alkohol und/oder Äther, vorzugsweise Tetrahydrofuran, mit UV-Licht bestrahlt, das Unlösliche abfiltriert, mit einem reduzierenden Mittel behandelt und mit einer Lösung von $H_2O_2$ in einem organischen Lösungsmittel, vorzugsweise Tetrahydrofuran oder einem tertiären aliphatischen Alkohol nachbehandelt. Diese kann z.B. erfolgen, indem man den Rückstand einige Tage in der in Beispiel 1b beschriebenen Oxidationslösung rührt. Die weitere Aufarbeitung erfolgt wie zuvor angegeben.

Das Ausmaß der nach diesen Ausführungsformen erzielbaren Beladung des Trägers durch Osmium ist innerhalb weiter Grenzen variierbar und nur durch die Zahl der koordinationsfähigen Stickstoffatome sowie durch die Zahl der durch $OsO_4$ oxidierbaren Zentren pro Formeleinheit begrenzt. Bei den Vinylpyridin-Polymeren (Formel $C_7H_7N$ der Masse 105,1 pro Monomereinheit) beträgt die maximale Beladungsdichte an Osmiumtetroxid (Molmasse 254,2) theoretisch 242 Gew.-% (2,42 kg $OsO_4$/kg Polymeres), wenn man nach einer vereinfachten Betrachtungsweise den Pyridinring als nur einmal komplexierbare Gruppe annimmt. Gewöhnlich arbeitet man mit erheblich geringerer Osmium-Beladung. Wenn man von Osmiumtetroxid ausgeht, arbeitet man vorzugsweise im Bereich von 5-200 Gramm Osmiumtetroxid auf 1 kg Polymeres, das sind 0,5 bis 20 Gew.-% oder 0,2 bis 8 mol-% (= 0,2 bis 8 % der theoretischen Maximalbeladung). Beispielsweise wird ein geeigneter erfindungsgemäßer Standardkatalysator mit 80 g OsO4 (oder 70 g $Os_3(CO)_{12}$) auf 1 kg Reillex 402 hergestellt; dies bedeutet, daß im Mittel auf etwa jede dreißigste Monomereinheit ein $OsO_x$-Fragment kommt.

Die Beladung der polymeren Träger mit $OsO_x$-Einheiten kann, wie im Beispiel 1 a angegeben, aus einer Lösung von $OsO_4$ in Tetrahydrofuran erfolgen. Bei der Einwirkung von Mengen unter etwa 20 Gew.-% $OsO_4$, bezogen auf das eingesetzte Poly(4-vinylpyridin), wird das gesamte in Lösung befindliche $OsO_4$ auf das Polymere aufgezogen. Dabei hängen Haltbarkeit und Qualität des Katalysators stark von der Dauer der chemischen Beladung ab, die in mechanistischer Betrachtungsweise im wesentlichen in mehreren Stufen abläuft:

1) Bildung einer Donor/Akzeptor-Bindung zwischen $OsO_4$ und der N-Base (z.B. Pyridin) des Polymeren. Solche Bindungen sind nach dem Stand der Literatur nicht sehr stark (vgl. M. Schröder, Chem. Rev. <u>80</u> -

(1980) 189), sondern lassen sich thermisch wieder spalten. Hält man das Herstellungsverfahren auf dieser Stufe an (nach etwa 3-5 Stunden), so sind die erhaltenen Katalysatoren bei Oxidationen von Olefinen mit $H_2O_2$ wenig selektiv.

2) Folgereaktionen durch einen Angriff des polymer gebundenen $OsO_4$ auf pyridinische Doppelbindungen. Erst dadurch kommt es zu einer sehr stabilen Bindung der oxidischen Osmium-Katalysatoren an die Polymermatrix

3) Das so erhaltene Pulver ist zwar bereits katalytisch aktiv. Zweckmäßig wird es aber mit $H_2O_2$ nachbehandelt, z.B. mit der in Beispiel 1b beschriebenen Oxidationslösung. Diese Maßnahme führt zur Blockierung der oxidationsempfindlichen Stickstoffatome, was eine bessere Reproduzierbarkeit von Aktivität und Selektivität des Katalysators bewirkt. Zugleich wird der Katalysator durch eine kontrollierte Oxidation geschützt.

Die Kombination der Reaktionen 1) und 2) war bisher nicht bekannt und findet auch bei der Arbeitsweise von Cainelli et al., l.c., nicht statt. Sie war auch nicht zu erwarten. Bis heute gilt nämlich die Lehrmeinung, daß sich die Komplexbildungsfähigkeit von Osmiumtetroxid auf Amin-Basen beschränkt (vgl. Cainelli et al., l.c.).

Wenn man die erfindungsgemäßen Heterogenkatalysatoren nach Filtration mehrere Stunden in einem Hochvakuum bei 28 ° C trocknet, wird kein Osmiumtetroxid freigesetzt. Selbst bei Temperaturen bis 300 ° C werden aus einem erfindungsgemäßen getrockneten Katalysator im Hochvakuum eines Massenspektrometers auch keine Spuren von Osmiumtetroxid oder anderen Osmiumoxiden freigesetzt. Ein für die Praxis (Beispiele 4 bis 27) empfehlenswerter Katalysator auf der Basis Reillex 402 (Beispiel 1) zeigt bis 300 ° C keine Anzeichen von Zerfall. Die erfindungsgemäße Osmium-Beladung polymerer Träger vermindert also die notorisch gefährliche Flüchtigkeit des freien Osmiumtetroxids zumindest soweit, daß selbst im Hochvakuum bei Raumtemperatur und bei üblichen Reaktionstemperaturen (bis ca. 150 ° C) keinerlei $OsO_4$-Austrag zu befürchten ist. Thermogravimetrische Untersuchungen im Temperaturbereich 25 bis 400 ° C (Beispiel 1c) ergeben auch für Atmosphärendruck ein vergleichbar positives Bild. Daß das Osmium in reduzierter, d.h. nicht mehr in achtwertiger Form vorliegt, zeigt sich auch daran, daß die Reaktion mit dem Polymeren bei Verwendung von $OsO_4$ dann erheblich beschleunigt wird, wenn man, wie oben ausgeführt, ein reduzierend wirkendes Mittel wie einen Alkohol zusetzt. Im IR-Spektrum ist die Bande von komplexiertem, aber intaktem $OsO_4$ verschunden. Im Gegenzug tauchen Schwingungen bei 846 $cm^{-1}$ (charakteristisch für trans-$OsO_2$-Einheiten in Os(VI)-Osmatestern) und 675 bzw. 647 $cm^{-1}$ auf. Letztere müssen verbrückenden Os-O-C-Einheiten zugeordnet werden, wie sie in Osmatestern des sechswertigen Osmiums auftreten.

Durch die beschriebene Verfahrensweise ist also erstmals eine Möglichkeit gefunden, in der das als Oxidationsmittel und Oxidationskatalysator viel verwendete Osmiumtetroxid zuverlässig und dauerhaft in eine ungiftige und dennoch katalytisch gut wirksame Form gebracht ist. Diese Verbindung liegt auf dem polymeren Träger nicht mehr als $OsO_4$ vor, sondern in einer nicht näher charakterisierbaren, mit Sicherheit aber reduzierten Form (wahrscheinlich als sechswertiges Osmium). Freies Osmiumtetroxid hingegen hat schon unter üblichen Bedingungen (25 ° C, 1 hPa) einen so großen Dampfdruck, daß es sich beim offenen Stehen an Luft rasch verflüchtigt.

Gleichermaßen sind mit der erfindungsgemäßen Verfahrensweise jetzt erstmals oxidische, osmiumhaltige, über Monate hinweg lagerfähige, luft- und wasserbeständige Heterogen-Katalysatoren verfügbar, die das Osmiumtetroxid hinsichtlich Aktivität und Selektivität streckenweise übertreffen.

Ein besonderer Vorteil der Erfindung besteht nämlich auch in der weitaus besseren Selektivität bei der Oxidation von olefinischen Bindungen zu Glykolen und insbesondere bei der stereospezifischen Oxidation von Olefinen, deren Doppelbindung sich in einem Ring befindet, zu cis-Diolen mit Hilfe des billigen, umweltfreundlichen Oxidationsmittels Wasserstoffperoxid; einen Vergleich zeigt Tabelle 1.

Die Anwendung der neuen Heterogenkatalysatoren erfolgt nicht wie jene des freien Osmiumtetroxids in Lösung (Homogenkatalyse), sondern in Form einer Heterogenkatalyse. Die in Wasser und organischen Lösungsmitteln unlöslichen Heterogen-Katalysatoren werden z.B. in einer Oxidationslösung suspendiert, wie oben und in Beispiel 1b ausgeführt. Diese enthält als wesentliche Bestandteile Wasserstoffperoxid, Wasser und ein geeignetes organisches Lösungsmittel, wie Tetrahydrofuran oder einen tertiären aliphatischen Alkohol, wie tert.-Pentanol, insbesondere aber tert.-Butanol. Geeignete andere Lösungsmittel sind z.B. Aceton, Methylenchlorid und Chloroform, wobei jedoch bei Verwendung von Aceton der Anteil des als Nebenprodukt aus dem jeweiligen Olefin gebildeten Ketols geringfügig zunimmt.

Die Figuren 1a bis 1c zeigen auch die Verschiedenheit der Spektren zwischen den erfindungsgemäßen Katalysatoren (Figuren 1b und 1c) und einem Katalysator, der nach der Arbeitsweise von Cainelli et al. hergestellt worden ist (Figur 1a). Gezeigt sind Ausschnitte aus den Spektren von Heterogenkatalysatoren, die durch Behandlung von Poly(4-vinylpyridin) mit Osmiumtetroxid in a) Cyclohexan, b) Tetrahydrofuran, c) Äthanol hergestellt wurden [Spektren nach graphischer Subtraktion des Ausgangspolymeren Poly(4-vinylpyridin)]. Die Spektren wurden mit einem FTIR-Spektrometer vom Typ Nicolet 740 mit einer diffusen Reflexionseinheit der Fa. Spectra-Tech. Inc. (Nr. 0030-005) aufgenommen.

Im Spektrum der Substanz a), hergestellt nach Cainelli et al., l.c., dominiert der peak von intaktem, an ein Stickstoffatom koordiniertem Osmiumtetroxid bei 907 $cm^{-1}$ (o). Diese Absorptionslinie ist typisch für Komplexe der Formel $OsO_4 \bullet L$, z.B. $OsO_4 \bullet$ Chinuclidin ($\nu$(OsO):925, 910, 900 cm $^{-}$). In den Spektren b) und c), die sich auf erfindungsgemäße Katalysatoren beziehen, ist dieser Peak verschwunden. Im Gegenzug tauchen jeweils zwei neue, intensive Banden bei 647 $cm^{-1}$ und 675 $cm^{-1}$ auf (x), die für Os-O-C-Schwingungen charakteristisch sind.

Tert.-Butylhydroperoxid, das sich nach Cainelli et al., l.c. als Oxidationsmittel sowohl in Kombination mit freiem als auch mit auf einem Träger fixiertem Osmiumtetroxid hervorragend als "primäres" Oxidationsmittel eignet, ist in Kombination mit den erfindungsgemäß vorgeschlagenen Katalysatoren unwirksam. Auch dieser Umstand belegt, daß in den erfindungsgemäßen Katalysatoren kein intaktes $OsO_4$ mehr vorliegt, sondern reduzierte Spezies wie $OsO_3$ oder, gegebenenfalls polymere, Oxide oder Oxoanionen.

Den erfindungsgemäßen Katalysatoren und deren Herstellung liegt nicht das Konzept zugrunde, daß diese Verbindungen stabile Komplexe mit basischen Aminen bildet. Während dieser Chemismus zwar Hilfsdienste für die anfängliche Verankerung der $OsO_4$-Moleküle auf den verwendeten Trägermaterialien leisten mag, verlaufen weitere, chemische, strukturverändernde Reaktionen während der Herstellung des Katalysators (Beispiel 1), was sich auch daran zeigt, daß sich abhängig von der Verfahrensweise auch das Osmium/Sauerstoff-Verhältnis verändert.

Bei der Anwendung der erfindungsgemäßen Katalysatoren ist zu beachten, daß diese neben den gewünschten Oxidationsreaktionen auch etwas die Zersetzung von $H_2O_2$ zu Sauerstoff und Wasser katalysieren, was aus der Gasentwicklung während der Reaktion ersichtlich ist. Daher muß eine überstöchiometrische Menge an $H_2O_2$ vorgelegt und ein geringer Verbrauch von Wasserstoffperoxid zur Reaktivierung des Katalysators in die Berechnung einbezogen werden. Die optimale Höhe des Überschusses läßt sich durch Vorversuche leicht ermitteln. In Extremfällen liegt das Verhältnis $H_2O_2$/Olefin bei 3:1. Das optimal einzustellende Verhältnis hängt von der Geschwindigkeit ab, mit der die Oxidation des Olefins abläuft, muß diesem also angepaßt werden. Im weiteren Verlauf der Oxidation erbringt dieser katalytische $H_2O_2$-Zerfall aber den Vorteil, daß vor der Isolierung des gebildeten Oxidationsproduktes die sonst übliche Vernichtung überschüssigen Oxidationsmittels, z.B. durch Zusatz von $MnO_2$, nur in geringem Maße erforderlich ist.

Die Dauer des Oxidationsprozesses ist eng mit der Reaktionstemperatur verknüpft. Die Oxidation wird in flüssiger Phase im allgemeinen bei -30 bis +100 °C durchgeführt, vorzugsweise bei 10 bis 70 °C. Wenn man bei relativ tiefen Temperaturen arbeiten will, kann die Zugabe eines Verdünners, wie Tetrahydrofuran, Aceton und/oder Methylenchlorid zweckmäßig sein. Bei Umsetzung elektronenarmer, desaktivierter Olefine empfiehlt es sich z.B., die Oxidation bei mindestens 50 °C, vorzugsweise bei 60 bis 70 °C durchzuführen. Die Reaktionszeiten verkürzen sich mit Temperaturerhöhung drastisch, und der Umsatz bei vorgegebener $H_2O_2$-Menge steigt. Dabei wirkt sich die erhöhte Temperatur nicht negativ auf die Selektivität der Reaktion aus, d.h. das Verhältnis zwischen vicinalem Diol und dem Folgeprodukt Ketol bleibt innerhalb enger Grenzen konstant. Temperaturerhöhung hat also fast keine Überoxidation zur Folge.

Ein weiterer Vorteil der Hydroxylierung von Olefinen mit den neuen Katalysatoren liegt in der Selbstindikation des Reaktionsendpunktes. Während der Oxidation wechselt die Farbe der Lösung von farblos nach orange bis braun. Nach Beendigung der Oxidation entfärbt sich die Reaktionsmischung weitgehend, und es ist zuletzt nur noch die gelbe Farbe des aktiven Katalysators zu sehen, der nun seinerseits wiederum "altert" und im Laufe von Tagen seine ursprüngliche braune Farbe zurückgewinnt.

Die Analyse des Katalysators nach mehrmaliger Anwendung zeigt, daß der Großteil der ursprünglichen Osmium-Menge noch vorhanden ist. Nur ein vernachlässigbarer Anteil ist in das katalytisch gewonnene Glykol eingeschleppt. Auch hierin liegt ein entscheidender Fortschritt gegenüber allen herkömmlichen, mit osmiumhaltigen oxidischen Katalysatoren arbeitenden Verfahren, bei denen mit ähnlichen Katalysatormengen gearbeitet, jedoch ein größerer Osmium-Anteil in das Oxidationsprodukt eingeschleppt wird, oder, um dieses zu vermeiden, in einem zusätzlichen Arbeitsschritt entfernt wird.

Der durch Abfiltrieren vom Reaktionsmedium und Trocknen im Vakuum der Ölpumpe bei Raumtemperatur auf einfache Weise wiedergewinnbare Katalysator kann erneut Anwendung finden, wobei die Aktivität im nachfolgenden Schritt nicht geringer ist. Der erfindungsgemäße Katalysator kann im diskontinuierlichen Betrieb also ohne weiteres mehrfach verwendet werden, auch für die Oxidation unterschiedlicher Olefine.

Die Erfindung bringt nicht nur den Vorteil mit sich, daß das Oxidationsprodukt praktisch frei von giftigen Osmiumverbindungen ist, sondern daß auch geringere Osmium-Mengen als bisher benötigt werden, was im Hinblick auf die hohen Osmiumpreise eine höhere Wirtschaftlichkeit bedeutet (1 g $OsO_4$ kostet z. Zt. 200 - 300 DM).

**I Beispiele 1 bis 3 - Herstellung von oxidischen, osmiumhaltigen Heterogenkatalysatoren**

1. Herstellung aus Osmiumtetroxid

a) 80 mg (0,31 mmol) Osmiumtetroxid (74,8 % Os) wurden in 30 ml absol. Tetrahydrofuran gelöst. Der Lösung wurden 1,00 g quervernetztes Poly(4-vinylpyridin) ("Reillex 402") zugegeben, das man vorher im Hochvakuum bei 80 °C (3 h) von anhaftendem Wasser befreit hatte. Es wurde dann magnetisch gerührt. Nach etwa 6 Stunden hatte der Niederschlag die ursprünglich gelbe Farbe der Lösung angenommen. Nach weiteren 66 h hatte sich das Polymere braun verfärbt. Der Feststoff wurde nach insgesamt dreitägigem Rühren abfiltriert, mit Tetrahydrofuran gewaschen und im Hochvakuum bei Raumtemperatur vom Lösungsmittel befreit. Geschieht die Isolierung des Katalysators bereits nach 3 h statt nach 72 h, so kann man das $OsO_4$ bei einer Temperatur von 110-140 °C bei einem Druck von etwa $10^{-5}$ Pa größtenteils wieder abspalten; hingegen ist bei Isolierung nach 3 Tagen Reaktionszeit beim angegebenen Druck (Hochvakuum) bis ca. 300 °C mit der empfindlichsten massenspektrometrischen Methode kein Austrag von $OsO_4$ nachweisbar, d.h. $OsO_4$ wird auch nicht in Spuren abgespalten.

Die Reaktionszeit konnte in Wiederholungsversuchen von 3 Tagen auf 2 Stunden abgekürzt werden, wenn man nach etwa einstündigem Rühren 1,5 bis 3 ml (26-52 mmol) Äthanol zugab.

| **Elementaranalysen:** | | |
|---|---|---|
| Berechnet für vollständigen $OsO_4$-Einbau: | Os 5,54 %; | O 1,87 % |
| Gefunden nach 3 Stunden Rührdauer: | Os 4,83 %; | O 3,41 % |
| Gefunden nach 3 Stunden Rührdauer: | Os 4,93 %; | O 4,67 % |
| ferner: C 73,08 %; H 6,63 %; N 11,69 % | | |

Die erhöhten Sauerstoff-Gehalte sind auf teilweisen Einbau von Sauerstoffatomen des Lösungsmittels Tetrahydrofuran zurückzuführen, da die Gehalte bei Verwendung von Methylenchlorid (1,9 %) statt Tetrahydrofuran nahe beim theoretischen Wert für den vollständigen $OsO_4$-Einbau (1,87 %) liegen. Diese Katalysatoren zeigen auch nicht die Wirksamkeit der erfindungsgemäßen Katalysatoren. Auch wenn man das Beispiel in $N_2$-Atmosphäre durchführt, also unter $O_2$-Ausschluß, sind die O-Gehalte größer als 3 %.

In analoger Weise wurden die in den Beispielen 26-42 verwendeten Katalysatoren hergestellt.

b) Das nach a) erhaltene braune Pulver wurde noch einen Tag lang in 20 ml einer "Oxidationslösung" gerührt, die wie folgt hergestellt worden war:

Zu 100 ml tert.-Butanol wurden 25 ml 30 %iges, handelsübliches $H_2O_2$ gegeben. Die Lösung wurde 1 h über wasserfreiem $MgSO_4$ gerührt. Dann wurde vom $MgSO_4$ abfiltriert. Die so hergestellte Oxidationslösung enthielt noch ca. 7 % Wasser.

Die Elementaranalyse des Katalysators nach Beladung mit 200 mg $OsO_4$ je 1,00 g des Polymeren und anschließender oxidativer Behandlung ergab folgende Werte: C 46,29 %; H 5,00 %; N 6,80 %; O 37,24 %; Os 4,67 %

Diese Zusammensetzung entspricht der Formel von Poly(4-vinylpyridin-N-oxid),das noch zusätzlich ca. 6 Gew.-% "Osmiumoxide" enthält. Zusätzlich ist noch ein Großteil der aromatischen Pyridin-Doppelbindungen (im Mittel 1,5 Doppelbindungsäquivalente pro Pyridinring) oxidiert. Der hohe Sauerstoffgehalt ist allein durch die Oxidation aller Doppelbindungen nicht erklärbar. Die Daten können je nach Beladungsdichte, Präparation und Lagerzeit des Katalysators um ±15 % relativ variieren.

c) **Thermogravimetrie**

Erhitzte man eine kleine Probe eines nach Beispiel 1b) bereiteten Katalysators auf einer Thermowaage (Fabrikat Perkin-Elmer) langsam (2,5 °C pro Minute) bis 350 °C, so blieb das Gewicht der Probe konstant, wenn man von geringen Wasserverlusten bei ca. 100 °C absieht. Ab 350 °C erfolgt rapider Gewichtsverlust bei gleichzeitiger Sublimation von Polymerbruchstücken an die gekühlten Teile des Ofens. Die Bindung des Osmiums in oxidischer Umgebung an den polymeren Träger ist also so stark, daß vor bzw. mit der Ablösung des Metalloxids auch das Polymergerüst zusammenbricht.

2. Herstellung eines Katalysators auf quervernetztem Poly(4-vinylpyridin-N-oxid)

6,00 g Reillex 402 wurden mit 70 ml der in Beispiel 1b beschriebenen "Oxidationslösung" übergossen und 12 Stunden bei Raumtemperatur magnetisch gerüht. Dann filtrierte man ab, wusch mit Tetrahydrofuran nach und trocknete den Rückstand 6 Stunden im Hochvakuum.

| Elementaranalyse für $C_7H_7NO$ (121,14): | | | | |
|---|---|---|---|---|
| Berechnet: | C 69,40 %, | H 5,82 %; | N 11,56 %; | O 13,20 % |
| Gefunden: | C 69,12 %, | H 5,60 %; | N 11,74 %; | O 13,40 % |

1,03 g des erhaltenen polymeren Pyridin-N-oxids wurden in 30 ml absol. Tetrahydrofuran suspendiert. Unter magnetischem Rühren gab man 200 mg (0,78 mmol) Osmiumtetroxid (74,8 % Os) in 5 ml desselben Lösungsmittels zu und rührte noch ca. 50 h bei Raumtemperatur. Zur Aufarbeitung des braunen Katalysators verfuhr man wie in Beispiel 1, Abschnitt a) und b) beschrieben.

| Elementaranalyse: | | | |
|---|---|---|---|
| C 61,31 %; | H 5,73 %; | N 9,03 %; | O 9,21 % |

3. Herstellung eines Katalysators aus Dodecacarbonyltriosmium

Eine Suspension von 1,50 g Reillex 402 in 70 ml Tetrahydrofuran (THF), in welcher 100 mg (0,11 mmol) $Os_3(CO)_{12}$ (Präparat der Fa. Strem Chemicals Inc., Newburyport, Mass., USA) gelöst waren, wurde ca. 48 h mit dem Licht einer Quecksilber-Hochdrucklampe (150 Watt) in einer gewöhnlichen Glasapparatur bestrahlt. Es erfolgte CO-Entwicklung. Dann filtrierte man ab, wusch mit THF nach und rührte den Rückstand 3 Tage in 60 ml der in Beispiel 1b beschriebenen "Oxidationslösung". Nach Abfiltrieren, Waschen mit THF, Behandeln mit einem reduzierenden Mittel wie Äthanol, nochmaligem Abfiltrieren und Waschen mit THF sowie Trocknen im Hochvakuum kann der Katalysator für Oxidationsreaktionen verwendet werden. Laut Elementaranalyse sind 94±4 % des eingesetzten Osmiums in das Polymere eingebaut.

**II Beispiele 4 bis 26 - Oxidation von olefinischen Verbindungen**

4-26) 5-10 ml der im Beispiel 1b beschriebenen Oxidationslösung wurden mit 30 mg des nach Beispiel 1b oder 2 hergestellten Katalysators versetzt. Die Menge an Oxidationslösung wurde in Abhängigkeit vom zu oxidierenden Alken gewählt. In der Regel wurden bei einer Reaktionstemperatur von 10 - 15°C (Wasserkühlung) 5 mmol des Alkens zugesetzt. Nach 3 h Reaktionszeit ließ man sich das Polymere absetzen, dekantierte die überstehende Lösung und wusch den Rückstand zwei Mal mit je 5 ml Tetrahydrofuran. Die vereinigten Lösungen wurden unter vermindertem Druck vom Lösungsmittel befreit. Das so erhaltene Rohprodukt war nach dreimaligem Waschen mit je 20 ml n-Hexan analysenrein. Falls notwendig, wurde das Reaktionsprodukt durch Destillation oder Umkristallisieren, gegebenenfalls bei erniedrigter Temperatur, aufgearbeitet. Der abfiltrierte Katalysator konnte erneut für weitere Katalyseansätze eingesetzt werden. Die untersuchten Olefin-Oxidationen sind mit den Resultaten in Tab. 1 (Katalysator nach Beispiel 1) und Tabelle 2 (Katalysator noch Beispiel 2) aufgelistet. Daraus geht hervor, daß mit dem neuen Verfahren in allen Fällen erheblich bessere Diol-Selektivitäten erzielt werden als mit den bekannten $OsO_4/H_2O_2$- und $OsO_4 \bullet$Polymer/$H_2O_2$-Verfahren.

In allen Versuchen mit den erfindungsgemäßen Katalysatoren wurde praktisch ein 100 %iger Umsatz erreicht. Die Ausbeuteangaben in den Tabellen 1 und 2 beziehen sich auf den Anteil des aufgeführten Reaktionsproduktes, bezogen auf die jeweils eingesetzte olefinische Verbindung. In Tabelle 1 sind die Angaben unter "Vergleich I" die Ausbeuten, welche im System $OsO_4/H_2O_2$ in tert.-Butanollösung erzielt wurden, also ohne Fixierung des Polymeren. Diese Daten wurden von M. Schröder, Chem. Rev. 80 (1980) 200-201, Tabelle III, übernommen. Vergleich II gibt das Ergebnis von eigenen Versuchen wieder. Dabei wurde ein Katalysator von $OsO_4$ auf quervernetztem Poly(4-vinylpyridin) analog Cainelli et al., l.c. verwendet, jedoch wurde statt mit tert.-Butylhydroperoxid mit $H_2O_2$ in tert.-Butanol gearbeitet und zwar unter den selben Bedingungen wie in den Beispielen 4 bis 26, also 3 Stunden bei 10-15°C. (Nach den Angaben der Autoren, die durch eigene Versuche bestätigt wurden, ist das Arbeiten mit tert.-Butylhydroperoxid als Primäroxidationsmittel dem mit $H_2O_2$ in Systemen von $OsO_4$ auf quervernetztem Poly(4-vinylpyridin) überlegen).

Die Stereochemie des Oxidationsprodukts von Olefinen, deren Doppelbindung sich in einem Ring befindet, kann nach Silylierung mit einem Gemisch aus Pyridin und Trimethylchlorsilan nach der Formelgleichung

$$\underset{OH\quad OH}{R^1R^2C - CR^3R^4} + (CH_3)_3SiCl/Pyridin \rightarrow \underset{(CH_3)_3SiO\quad OSi(CH_3)_3}{R^1R^2C - CR^3R^4}$$

ermittelt werden. Untersuchungen mit einem Gaschromatographen HP 5890A mit MSD 5970B und dem Rechnersystem HP 9000-300 an einer Kapillarsäule, bestehend aus Methylpolysiloxan mit 5 % Phenylpolysiloxan ergaben, daß nach dieser Vorbehandlung die Aufspaltung von cis- und trans-Stereoisomeren gelingt. Beim Vergleich mit authentischen Proben zeigte sich, daß die erfindungsgemäß nach den Beispielen 4 bis 6, 8, 23, 28 und 29 erhaltenen cyclischen Reaktionsprodukte zu über 99 % cis-Konfiguration besitzen.

**Tabelle 1:** Oxidation von olefinischen Verbindungen an Katalysatoren nach Beispiel 1b

| Beispiel | Ausgangsmaterial | Produkt | Ausbeute | Vergleich I | Vergleich II |
|---|---|---|---|---|---|
| 4 | Cyclohexen | 1,2-Dihydroxycyclohexan | 76 % | 58 % | 66 % |
| 5 | Cycloocten | 1,2-Dihydroxycyclooctan | 99 % | 11 % | |
| 6 | Cyclododecen | 1,2-Dihydroxycyclododecan | 64 % | | |
| 7 | Styrol | 1,2-Dihydroxyphenyläthan | 75 % | 50 % | 0 % |
| 8 | Cyclooctatetraen | 1,2-Dihydroxy-3,5,7-cyclooctatrien | 45 % | | |
| 9 | 1-Octen | 1,2-Dihydroxyoctan | 73 % | | 33 % |
| 10 | trans-2-Octen | 2,3-Dihydroxyoctan | 65 % | | |
| 11 | trans-4-Octen | 4,5-Dihydroxyoctan | 100 % | | |
| 12 | Ölsäuremethylester | 9,10-Dihydroxyundecansäuremethylester | 92 % | 60 % | |
| 13 | Allylalkohol | Glycerin | 80 % | 60 % | 0 % |
| 14 | 1-Decen | 1,2-Dihydroxydecan | 80 % | | 31 % |
| 15 | cis-Stilben | 1,2-Dihydroxy-1,2-diphenyläthan | 75 % | | |
| 16 | 2,3,4,5,6-Pentafluorstyrol | 1-(2,3,4,5,6-Pentafluorphenyl)-1,2-dihydroxyäthan | 85 % | | |
| 17 | Crotonsäureäthylester | 2,3-Dihydroxybuttersäureäthylester | 95 % | 56 % | |
| 18 | Methacrylsäure-n-butylester | 2,3-Dihydroxy-2-methylpropionsäure-n-butylester | 100 % | | 14 % |
| 19 | Maleinsäurediäthylester | meso-Weinsäurediäthylester | 71 %°) | 41 % | |
| 20 | Mesityloxid | 3,4-Dihydroxy-2-pentanon | 92 % | 23 % | 0 % |
| 21 | Methylencyclobutan | 1-Hydroxy-1-hydroxymethyl-cyclobutan | 100 % | 39 % | |
| 22 | Allylbromid | 1-Brom-2,3-dihydroxypropan | 84 % | 13 % | |

°) Reaktionstemperatur 60°C

Tabelle 2

| Oxidation von olefinischen Verbindungen an Katalysatoren nach Beispiel 2 (Oxidationsbedingungen wie im Beispiel 1) | | | |
|---|---|---|---|
| Beispiel | Ausgangsmaterial | Produkt | Ausbeute |
| 23 | Cyclohexen | 1,2-Dihydroxycyclohexan (cis-spezifisch) | 81 % |
| 24 | Allylalkohol | Glycerin | 90 % |
| 25 | 1-Decen | 1,2-Dihydroxydecan | 88 % |
| 26 | trans-4-Octen | 4,5-Dihydroxyoctan | 95 % |

**Beispiel 27 - Oxidation von Methacrylsäure-n-butylester**

In einem 250 ml-Rundkolben wurden 60 ml der im Beispiel 1b beschriebenen Oxidationslösung vorgelegt, die dann bei 0 °C mit 50 mg des Katalysators nach Beispiel 1b versetzt wurde. Bei einer Temperatur von 0 °C (Eiskühlung) wurden im Verlaufe einer Stunde 9,4 ml (60 mmol) Methacrylsäure-n-butylester zugetropft. Man ließ die Suspension innerhalb einer Stunde auf Raumtemperatur erwärmen und rührte weitere 120 Minuten. Dann wurde das Polymere durch Filtration abgetrennt und das Filtrat vorsorglich mit 20 mg $MnO_2$ versetzt. Nach 30 Minuten wurde abfiltriert und das Lösungsmittel aus dem klaren Filtrat im Vakuum einer Ölpumpe abgedampft. Die Destillation des Rückstandes unter vermindertem Druck (1 Pa) ergab 2,3-Dihydroxy-2-methylpropionsäure-n-butylester in analysenreiner Form mit einer Siedetemperatur von 105 °C (1 Pa). Die Ausbeute nach der Destillation betrug 90 % (9,36 g).

**Beispiele 28 - 45 - Oxidation von olefinischen Verbindungen**

Tabelle 3 zeigt Oxidationsansätze mit den unter $i_3$) und $i_4$) beschriebenen Katalysatoren. Die Reaktionen wurden unter den für die Beispiele 4 bis 26 genannten Bedingungen unter Einsatz der in Beispiel 1b beschriebenen Oxidationslösung durchgeführt. Die Ausbeuten sind auf die umgesetzte Menge des Ausgangsstoffs bezogen, wobei der Umsatz 100 % betrug.

Tabelle 3

| Oxidation von olefinischen Verbindungen an siliziumhaltigen Katalysatoren | | | | |
|---|---|---|---|---|
| Beispiel | Ausgangsmaterial | Produkt | Ausbeute | |
| | | | $i_3$) | $i_4$) |
| 28 + 29 | Cyclohexen | 1,2-Dihydroxycyclohexan | 90 % | 87 % |
| 30 + 31 | Styrol | 1,2-Dihydroxyphenyläthan | 53 % | 46 % |
| 32 + 33 | 1-Octen | 1,2-Dihydroxyoctan | 55 % | 50 % |
| 34 + 35 | trans-2-Octen | 2,3-Dihydroxyoctan | 70 % | 70 % |
| 36 + 37 | 1-Decen | 1,2-Dihydroxydecan | 56 % | 48 % |
| 39 + 39 | Crotonsäureäthylester | 2,3-Dihydroxybuttersäureäthylester | 76 % | 75 % |
| 40 + 41 | Methacrylsäure-n-butylester | 2,3-Dihydroxy-2-methylpropionsäure-n-butylester | 89 % | 90 % |
| 42 + 43 | Maleinsäurediäthylester | meso-Weinsäurediäthylester | 70 % | 74 % |
| 44 + 45 | Mesityloxid | 3,4-Dihydroxy-2-pentanon | 81 % | 84 % |

**Patentansprüche**

1. Oxidische Heterogenkatalysatoren auf der Grundlage von stickstoffhaltigen Polymeren, an die oxidisches Osmium in einer niedrigeren als der achtwertigen Oxidationsstufe gebunden ist.

2. Verfahren zur Herstellung von Katalysatoren auf der Grundlage von stickstoffhaltigen Polymeren, auf die ein Osmiumoxid aus einem Lösungsmittel niedergeschlagen ist, dadurch gekennzeichnet, daß man ein in Alkoholen und/oder Äthern lösliches Osmiumoxid in Gegenwart eines solchen Alkohols und/oder

Äthers mit einem in Wasser und dem angewandten Alkohol und Äther unlöslichen Polymeren, das Stickstoff in offenkettiger, heterocyclischer und/oder Amidbindung bzw. ein davon abgeleitetes N-Oxid enthält, unter Bedingungen, unter denen die achtwertige Oxidationsstufe des Osmiums nicht beständig ist, umsetzt und den oxidisches Osmium enthaltenden Katalysator abtrennt und trocknet.

**3.** Verfahren nach Anspruch 2, dadurch gekennzeichnet, daß man 5 bis 200 g Osmiumtetroxid auf 1 kg Polymeres verwendet.

**4.** Verfahren nach Anspruch 2 oder 3, dadurch gekennzeichnet, daß man den Katalysator mit einer Lösung von $H_2O_2$ in einem organischen Lösungsmittel, vorzugsweise Tetrahydrofuran oder einem tertiären aliphatischen Alkohol, nachbehandelt.

**5.** Verfahren zur Herstellung von Katalysatoren auf der Grundlage von stickstoffhaltigen Polymeren, auf die eine Osmiumverbindung aus einem Lösungsmittel niedergeschlagen ist, dadurch gekennzeichnet, daß man eine Osmium-Carbonyl-Verbindung in einem Alkohol und/oder Äther mit einem in Wasser und dem angewandten Alkohol und Äther unlöslichen Polymeren, das Stickstoff in offenkettiger, heterocyclischer und/oder Amidbindung bzw. ein davon abgeleitetes N-Oxid enthält, unter Bedingungen umsetzt, die zu einer Fixierung eines Osmiumcarbonyls oder eines CO-haltigen Bruchstücks der Carbonylosmium-Verbindung auf dem Polymeren führt und das in dem so erhaltenen Produkt enthaltene Osmium mit einer Peroxyverbindung, insbesondere $H_2O_2$ zu einer höheren, jedoch nicht der achtwertigen Oxidationsstufe oxidiert.

**6.** Verfahren nach Anspruch 5, dadurch gekennzeichnet, daß man eine Suspension des betreffenden Polymeren in einer Lösung von Dodecacarbonyltriosmium in einem Alkohol und/oder Äther mit UV-Licht bestrahlt, das Unlösliche abfiltriert, mit einem reduzierenden Mittel behandelt und mit einer Lösung von $H_2O_2$ in einem organischen Lösungsmittel, vorzugsweise Tetrahydrofuran oder einem tertiären aliphatischen Alkohol, nachbehandelt.

**7.** Verfahren nach einem oder mehreren der Ansprüche 2 bis 6, dadurch gekennzeichnet, daß man die Reaktion in einem Äther und/oder einem Alkohol mit mehr als 3 C-Atomen durchführt und nach teilweisem Ablauf der Reaktion ein reduzierendes Mittel zugibt, vorzugsweise einen einwertigen aliphatischen Alkohol mit 1 bis 3 C-Atomen, insbesondere Methanol oder Äthanol.

**8.** Verfahren nach Anspruch 7, dadurch gekennzeichnet, daß der Alkohol in einer Menge von 2 bis 20, vorzugsweise von 3 bis 6 Mol je Mol $OsO_4$ zugesetzt wird.

**9.** Verwendung eines Katalysators nach Anspruch 1 zur Überführung von olefinischen Bindungen in vicinale Diole.

**10.** Verfahren zur Hydroxylierung von olefinischen Bindungen zu Glykolen durch Einwirkung von Peroxiden, dadurch gekennzeichnet, daß man olefinische Verbindungen der Formel $R^1R^2C=CR^3R^4$, in denen $R^1$ bis $R^4$ gleich oder verschieden sind, wobei einer oder zwei dieser Reste aromatisch sein können, die übrigen aber nicht-aromatische Reste bedeuten, mit Wasserstoffperoxid, das in einem von aliphatischen Kohlenwasserstoffen verschiedenen Lösungsmittel gelöst ist, an oxidischen Heterogenkatalysatoren nach Anspruch 1 umsetzt.

**11.** Verfahren nach Anspruch 10, dadurch gekennzeichnet, daß wenigstens der Rest $R^1$ Wasserstoff darstellt.

**12.** Verfahren nach Anspruch 10 oder 11, dadurch gekennzeichnet, daß Olefine, die außer der zu oxidierenden Doppelbindung Gruppen enthalten, die die freie Drehbarkeit beeinträchtigen, in cis-vicinale Diole übergeführt werden.

**13.** Verfahren nach einem oder mehreren der Ansprüche 10 bis 12, dadurch gekennzeichnet, daß die Oxidation bei -30 bis $+100\,^\circ C$, vorzugsweise bei $+10$ bis $70\,^\circ C$ durchgeführt wird.

## Claims

1. An oxidic heterogeneous catalyst based on a nitrogen-containing polymer, to which oxidic osmium is bound in a lower oxidation state than the octavalent oxidation state.

2. A process for preparing a catalyst based on a nitrogen-containing polymer, onto which an osmium oxide has been precipitated from a solvent, which comprises reacting an osmium oxide, which is soluble in alcohols and/or ethers, in the presence of such an alcohol and/or ether with a polymer, which is insoluble in water and in the alcohol and ether used and which contains nitrogen in an open-chain bond, heterocyclic bond and/or amide bond or an N-oxide derived therefrom, under conditions under which the octavalent oxidation state of osmium is unstable, and separating off and drying the catalyst which contains oxidic osmium.

3. The process as claimed in claim 2, wherein 5 to 200 g of osmium tetroxide are used per 1 kg of polymer.

4. The process as claimed in claim 2 or 3, wherein the catalyst is aftertreated with a solution of $H_2O_2$ in an organic solvent, preferably tetrahydrofuran or a tertiary aliphatic alcohol.

5. A process for preparing a catalyst based on a nitrogen-containing polymer, onto which an osmium compound has been precipitated from a solvent, which comprises reacting an osmium carbonyl compound in an alcohol and/or ether with a polymer, which is insoluble in water and in the alcohol and ether used and which contains nitrogen in an open-chain bond, heterocyclic bond and/or amide bond or an N-oxide derived therefrom, under conditions which lead to a fixation of an osmium carbonyl or of a CO-containing fragment of the carbonyl-osmium compound on the polymer, and oxidizing the osmium contained in the product thus obtained with a peroxy compound, especially $H_2O_2$, to a higher oxidation state but not to the octavalent oxidation state.

6. The process as claimed in claim 5, wherein a suspension of the particular polymer in a solution of dodeca-carbonyl-triosmium in an alcohol and/or ether is irradiated with UV light, and the insoluble matter is filtered off, treated with a reducing agent and after-treated with a solution of $H_2O_2$ in an organic solvent, preferably tetrahydrofuran or a tertiary aliphatic alcohol.

7. The process as claimed in one or more of claims 2 to 6, wherein the reaction is carried out in an ether and/or an alcohol having more than 3 carbon atoms and, after the reaction has partially proceeded, a reducing agent is added, preferably a monohydric aliphatic alcohol having 1 to 3 carbon atoms, especially methanol or ethanol.

8. The process as claimed in claim 7, wherein the alcohol is added in a quantity of from 2 to 20 and preferably from 3 to 6 mol per mol of $OsO_4$.

9. The use of a catalyst as claimed in claim 1 for converting an olefinic bond into a vicinal diol.

10. A process for hydroxylating an olefinic bond to a glycol by the action of a peroxide, which comprises reacting an olefinic compound of the formula $R^1R^2C=CR^3R^4$, in which $R^1$ to $R^4$ are identical or different and one or two of these radicals can be aromatic but the others are non-aromatic radicals, with hydrogen peroxide dissolved in a solvent other than an aliphatic hydrocarbon, over an oxidic heterogeneous catalyst as claimed in claim 1.

11. The process as claimed in claim 10, wherein at least the radical $R^1$ is hydrogen.

12. The process as claimed in claim 10 or 11, wherein an olefin which, in addition to the double bond to be oxidized, contains groups which hinder free rotation, is converted into a cis-vicinal diol.

13. The process as claimed in one or more of claims 10 to 12, wherein the oxidation is carried out at -30 to +100 °C, preferably at +10 to 70 °C.

## Revendications

1. Catalyseurs hétérogènes d'oxydation sur un support de base en des polymères contenant de l'azote, sur lesquels est fixé de l'osmium oxydé à un degré d'oxydation inférieur au degré huit (inférieur à l'octavalence).

2. Procédé de préparation de catalyseurs sur un support de base en des polymères contenant de l'azote, sur lesquels est précipité à partir d'un solvant un tétroxyde d'osmium, procédé caractérisé en ce qu'on fait réagir un tétroxyde d'osmium, soluble dans des alcools et/ou dans des éthers, en présence d'un tel alcool ou d'un éther avec un polymère insoluble dans l'eau et insoluble dans l'alcool et l'éther que l'on utilise, ce polymère contenant de l'azote dans une chaîne ouverte, dans un hétérocycle et/ou dans une liaison ou une chaînon amide ou dans un N-oxyde qui en dérive, en opérant dans des conditions dans lesquelles le degré huit d'oxydation de l'osmium n'est pas stable et l'on sépare et sèche le catalyseur contenant de l'osmium oxydé.

3. Procédé selon la revendication 2, caractérisé en ce qu'on utilise 5 à 200 g de tétroxyde d'osmium pour 1 kg du polymère.

4. Procédé selon la revendication 2 ou 3, caractérisé en ce qu'on traite ensuite le catalyseur dans une solution de $H_2O_2$ dans un solvant organique, avantageusement le tétrahydrofuranne, ou dans un alcool aliphatique tertiaire.

5. Procédé pour préparer des catalyseurs sur un support de base formé par des polymères contenant de l'azote, sur lesquels un composé d'osmium est précipité à partir d'un solvant, procédé caractérisé en ce qu'on fait réagir un composé de type osmium-carbonyle dans un alcool et/ou un éther avec un polymère insoluble dans l'eau et dans l'alcool et l'éther que l'on utilise, ce polymère contenant l'azote en chaîne ouverte, dans un hétérocycle et/ou dans un terme de liaison ou chaînon amide ou un N-oxyde qui en dérive, en opérant dans des conditions conduisant à une fixation d'un osmium-carbonyle ou d'un fragment, contenant CO, de l'osmium-carbonyle sur le polymère, et l'on oxyde l'osmium contenu dans le produit ainsi obtenu, en utilisant un composé peroxydé, notamment $H_2O_2$, jusqu'à un degré supérieur d'oxydation mais qui n'est pas le degré huit d'oxydation (l'octavalence).

6. Procédé selon la revendication 5, caractérisé en ce qu'on soumet une suspension du polymère en cause dans une solution de dodécacarbonyle triosmium dans un alcool et/ou dans un éther à une irradiation par de la lumière ultra-violette, on sépare par filtration la partie insoluble, on la traite par un agent réducteur et par une solution de $H_2O_2$ dans un solvant organique, avantageusement le tétrahydrofuranne ou un alcool aliphatique tertiaire.

7. Procédé selon une ou plusieurs des revendications 2 à 6, caractérisé en ce qu'on effectue la réaction dans un éther et/ou dans un alcool comportant plus de trois atomes de carbone et, après déroulement partiel de la réaction, on ajoute un agent réducteur, avantageusement un monoalcool aliphatique comportant 1 à 3 atomes de C, notamment le méthanol ou l'éthanol.

8. Procédé selon la revendication 7, caractérisé en ce qu'on ajoute l'alcool en une quantité de 2 à 20, avantageusement de 3 à 6 moles par mole de $OsO_4$

9. Utilisation d'un catalyseur selon la revendication 1 pour transformer des liaisons oléfiniques en des diols vicinaux.

10. Procédé d'hydroxylation de liaisons oléfiniques en des glycols par l'action de peroxydes, procédé caractérisé en ce qu'on fait réagir des composés oléfiniques de formule $R^1R^2C=CR^3R^4$ (dans laquelle les symboles $R^1$ à $R^4$ sont identiques ou différents, un ou deux ces restes pouvant être aromatiques, mais les autres étant des restes non aromatiques) avec du peroxyde d'hydrogène qui est dissous dans un solvant différant d'hydrocarbures aliphatiques, sur des catalyseurs hétérogènes d'oxydation selon la revendication 1.

11. Procédé selon la revendication 10, caractérisé en ce qu'au moins le symbole $R^1$ représente un atome d'hydrogène.

**12.** Procédé selon la revendication 10 ou 11, caractérisé en ce qu'on transforme en des diols vicinaux cis des oléfines qui, en plus de la double liaison à oxyder, contiennent des groupes gênant la possibilité de rotation libre.

**13.** Procédé selon une ou plusieurs des revendications 10 à 12, caractérisé en ce qu'on effectue l'oxydation entre -30 et + 100 °C, avantageusement entre + 10 et + 70 °C.

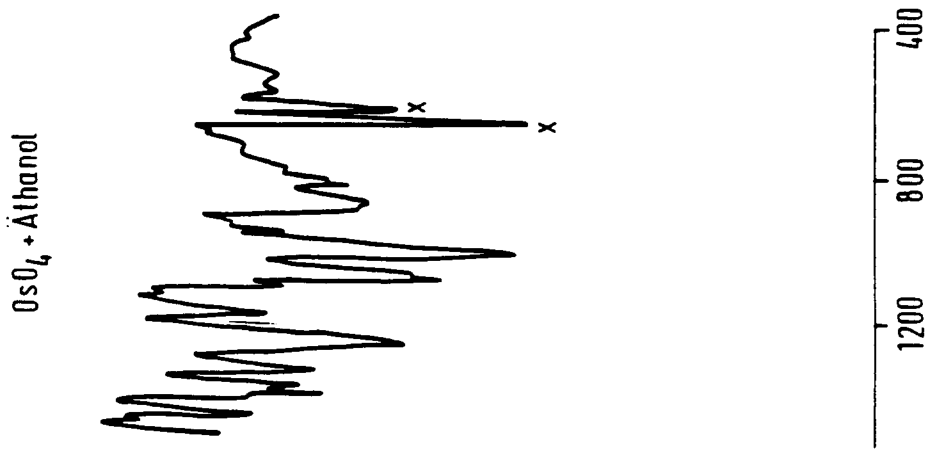


Fig. 1c

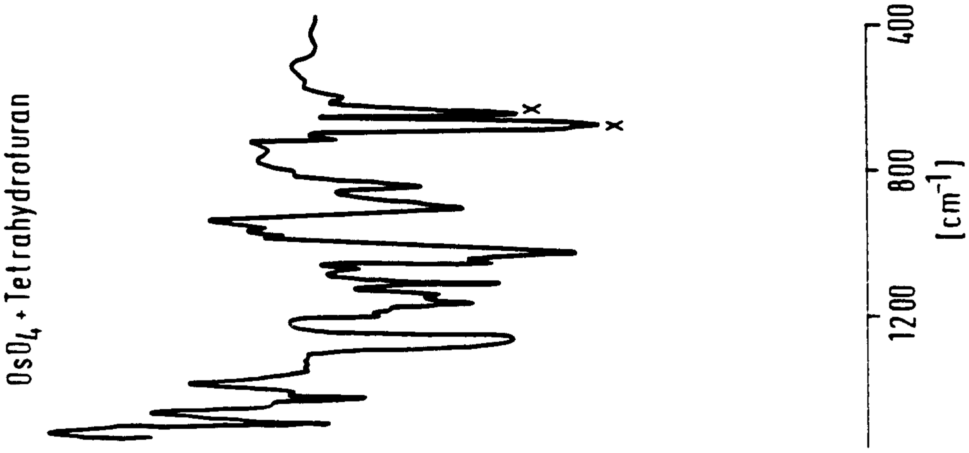


Fig. 1b

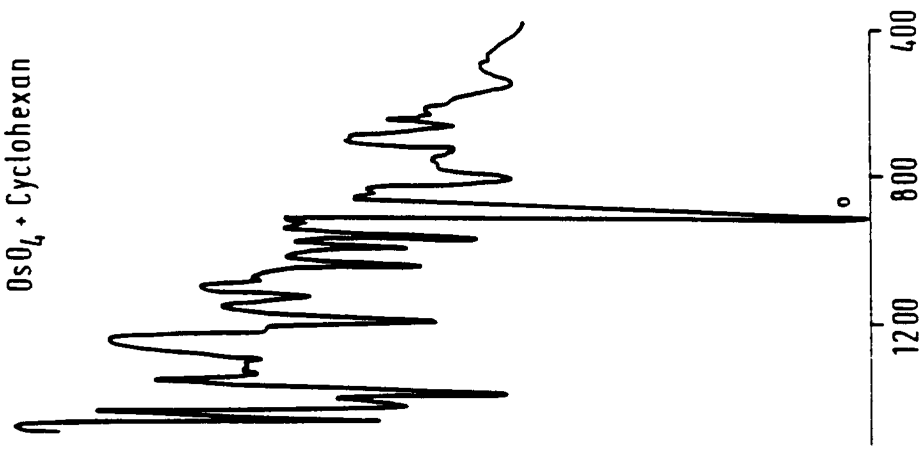


Fig. 1a